# EUROPEAN PATENT SPECIFICATION

(11) **EP 0 709 675 B1**
(45) Date of publication and mention of the grant of the patent: **13.03.2002**
(21) Application number: 95307606.4
(22) Date of filing: 25.10.1995
(51) Int. Cl.: G01N 33/52, G01N 33/558

(54) **Chromatogenous testing system for urinalysis**
Chromatogenes Testsystem zur Harnanalyse
Système d'essai chromatogénique pour l'analyse d'urine

(30) Priority: 29.10.1994 JP 1499594
(43) Date of publication of application: 01.05.1996
(73) Proprietor: KABUSHIKI KAISHA NIPPON GENE, Tokyo 930 (JP)
(72) Inventor: Yoneda Yuko, c/o Kabushiki Kaisha Nippon Gene, Tokyo 930 (JP); Itooka, Toshiyuki c/o Kabushiki Kaisha Nippon Gene, Tokyo 930 (JP)
(74) Representative: Thornley, Rachel Mary

(56) References cited:
- EP-A- 0 046 004
- EP-A- 0 421 294
- EP-A- 0 525 829
- WO-A-86/04683
- WO-A-92/01226
- WO-A-94/24563
- US-A- 4 631 174

## Description

The present application relates to a chromatogenous testing system for urinalysis, in which both sides and upper surface of a reagent phase and a urine-reagent developing phase as well as both sides of a support member where the reagent phase and the urine-reagent . developing phase are attached are waterproof so that development of chromatogenous solvent may proceed unhindered even when the urine to be tested is directly applied on sectors where the chromatogenous solvent is developing.

In a conventional type chromatogenous testing system for urinalysis, after the urine to be tested is collected into a container such as a urine cup, only a urine applying sector of the chromatogenous testing system is immersed in the urine or the urine to be tested is taken into a syringe, and it is then applied on the urine applying sector.

WO-A-86/04683 discloses a strip-format test device for determination of clinical parameters in a liquid sample in which the sample has to be carefully disposed on the reagent phase through a hole made on the support member. WO-A-94/24563 discloses a completely sealed system, whereby the reagent phase is enclosed by two non-wettable double-sided adhesive tapes, which serve as fixing material for two sealing plastic sheets. The sample can only be introduced in the reagent phase by opening temporarily the system sealing.

In the conventional type chromatogenous testing system as described above, when the urine specimen is placed on the urine applying sector at one end of the system, the urine to be tested often splashes, and the splashed urine may then directly invade the developing sector of the device (hereinafter referred as "urine-reagent" sector because the urine is mixed here with the reagent in the system during development). Urine advances by diffusion and develops continuously from the urine applying sector to the developing phase through a reagent phase, carrying the reagent into the developing sector. As a result of splashes, the development of the urine-reagent from the urine applying sector is hindered, and it is not always possible to obtain accurate reaction results.

Some of the known testing systems are designed as waterproof, but it is not possible to prevent infiltration of the urine to be tested from both sides, and accurate reaction results cannot be obtained.

For this reason, the urine to be tested must be put into the urine testing unit using a urine cup or a syringe, and this can be a troublesome procedure.

International patent application WO-A-86/04683 discloses a strip-format test device for determination of clinical parameters in a liquid sample. However, this device also suffers from the disadvantages described above.

International patent application WO-A-94/24563 describes the use of an assay device for detecting the presence of analyte in a sample. The device comprises a nylon membrane sealed between transparent adhesive tape and a stiff plastic strip. Although the system described is sealed, it requires the opening of the sealing system for the sample to be introduced to the reagent phase.

It is an object of the present invention to provide a testing system, by the use of which such troublesome procedure may be eliminated and the adverse effect of the splashed urine when the urine is put to the urine applying sector is avoidable.

In the apparatus of the present invention, the development of the urine reagent is not hindered by splashed urine even when the urine to be tested is directly put to the urine applying sector and more accurate reaction results can be obtained.

Thus, in accordance with the present invention, there is provided a chromatogenous testing system for urinalysis comprising a support member, a reagent phase and a development phase having a urine-reagent developing sector, wherein the edges and upper surface of the reagent phase and of the urine-reagent developing sector as well as of the portion of the support member to which said phase and said sector are attached, are accommodated in a cylindrical or envelope-like shaped transparent resin film having thermal contractibility.

In the following, a more detailed description will be given, by way of example only, of preferred embodiments of the invention, referring to the drawings, in which
Fig. 1 is a perspective view of a conventional type chromatogenous testing system;
Fig. 2 is a perspective view of a first embodiment of the present invention; and

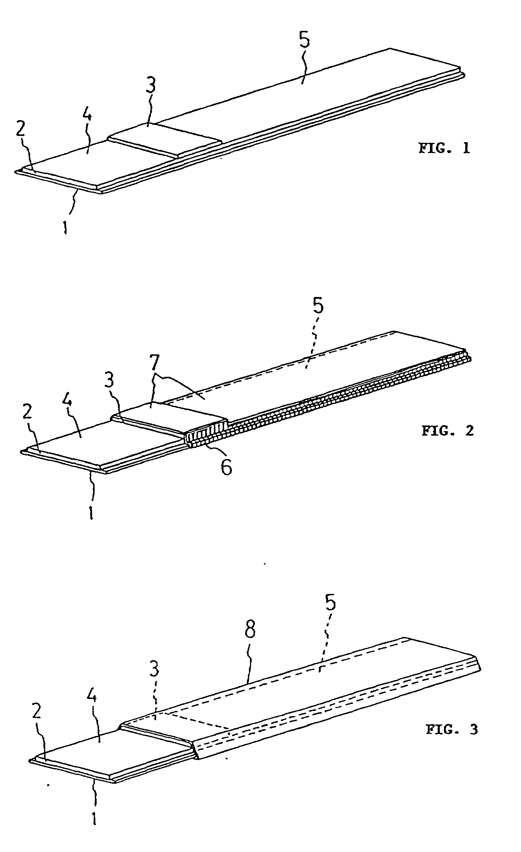

Fig. 2 is a perspective view of a second embodiment of the present invention.

In the figures, reference numeral 1 represents a support member, 2 represents a developing phase, 3 a reagent phase, 4 urine applying sector, 5 a urine-reagent developing phase, 6 a water repellent agent, 7 a transparent resin film, and 8 represents an envelope-like or cylindrical transparent resin film or container.

Fig. 1 is a perspective view of a conventional type chromatogenous testing system, in which a developing phase (2) is provided on upper surface of a support member (1), and a reagent phase (3) is positioned on a sector closer to a first end of the developing phase (2). With the reagent phase (3) as a border, a urine applying sector (4) is positioned in the short sector closer to the first end of the developing phase (2), and the remaining long sector is used as the urine-reagent developing phase (5). The urine applying sector (4) is immersed in the urine to be tested which has been collected in a urine cup or the urine to be tested sucked into a syringe is put onto the urine applying sector (4) to perform the test.

### (Example 1)

Next, description will be given on Example 1 of the chromatogenous testing system of the present utility model, referring to the perspective view of Fig. 2. As an addition to the conventional type testing system device of Fig. 1, a water repellent agent (6) is coated on both sides of the reagent phase (3) and the urine-reagent developing phase (5) as well as on both sides of the support member (1) where the reagent phase (3) and the urine-reagent developing phase (5) are provided. Then, the water repellent agent is solidified by irradiating heat, ultraviolet ray, infrared ray, etc., and a transparent resin film (7) is attached on upper surfaces of the reagent phase (3) and the urine-reagent developing phase (5).

### (Example 2)

Example 2 of the chromatogenous testing system of the present utility model is described in connection with the perspective view of Fig. 3. As an addition to the conventional type testing system device of Fig. 1, the support member (1) with the reagent phase (3) and the urine-reagent developing phase (5) provided on it is accommodated in an envelope-like or cylindrical transparent resin film or container (8).

If the envelope-like or cylindrical transparent resin film or container (8) is made of a shrinkable resin, which is shrunk by heating, and the reagent phase (3) and the urine-reagent developing phase (5) as well as the support member (1) with these two phases are accommodated in the envelope-like or cylindrical transparent resin film or container (8) and if these are heated and shrunk, there will be no possibility to drop off.

Because the testing system of the present utility model is designed as described above, even when the urine to be tested is directly applied on the urine applying sector (4), splashed urine is repelled by the water repellent agent (6) and transparent resin film (7) or by the envelope-like or cylindrical transparent resin film or container (8), and the urine is not infiltrated to the reagent phase (3) or the urine-reagent developing phase (5). As a result, the urine-reagent in the urine-reagent developing phase(5) is normally developed and more accurate reaction results can be obtained. Also, troublesome procedure to use urine cup or syringe can be avoided.

## Claims

1. A chromotogenous testing system for urinalysis comprising a support member, a reagent phase (3) and a development phase (5) having a urine-reagent developing sector, wherein the edges and upper surface of the reagent phase and of the urine-reagent developing sector as well as of the portion of the support member to which said phase and said sector are attached, are accommodated in a cylindrical or envelope-like shaped transparent resin film having thermal contractibility.

2. A chromotagenous testing system for urinalysis according to claim 1 wherein said transparent resin film is an envelope-like transparent resin film.

3. A chromotagenous testing system for urinalysis according to claim 1 wherein said transparent resin film is a cylindrical transparent resin film.

## Patentansprüche

1. Chromatogenes Testsystem zur Harn-Analyse, umfassend ein Trägerelement, eine Reagensphase (3) und eine Entwicklungsphase (5), die einen Harn-Reagensentwicklungssektor aufweist, worin die Ränder und die Deckfläche der Reagensphase und des Harn-Reagensentwicklungssektors sowie der Abschnitt des Trägerelements, an den die Phase und der Sektor gebunden sind, in einer wärmeschrumpfbaren transparenten Harzfolie mit zylindrischer oder hüllenartiger Form untergebracht sind.

2. Chromatogenes Testsystem zur Harn-Analyse nach Anspruch 1, worin die transparente Harzfolie eine hüllenartige transparente Harzfolie ist.

3. Chromatogenes Testsystem zur Harn-Analyse nach Anspruch 1, worin die transparente Harzfolie eine zylindrische transparente Harzfolie ist.

## Revendications

1. Un système d'essai chromatogénique pour analyse d'urine comprenant un organe de support, une phase réactive (3) et une phase de développement (5) présentant un secteur de développement réactif à l'urine, dans lequel les bords et la surface supérieure de la phase réactive et du secteur de développement réactif à l'urine, ainsi que la partie de l'organe de support auquel sont fixés ladite phase et ledit secteur, sont logés dans une pellicule de résine transparente cylindrique ou en forme d'enveloppe et présentant une contractibilité thermique.

2. Un système d'essai chromatogénique pour analyse d'urine selon la revendication 1 dans lequel ladite pellicule de résine transparente est une pellicule de résine transparente en forme d'enveloppe.

3. Un système d'essai chromatogénique pour analyse d'urine selon la revendication 1 dans lequel ladite pellicule de résine transparente est une pellicule de résine transparente cylindrique.
